# EUROPEAN PATENT SPECIFICATION

(11) **EP 2 726 030 B1**
(45) Date of publication and mention of the grant of the patent: **12.08.2015**
(21) Application number: 12732777.3
(22) Date of filing: 28.06.2012
(51) Int. Cl.: A61F 5/443, A61F 5/449

(54) **AN OSTOMY APPLIANCE**
KÜNSTLICHER DARMAUSGANG
ACCESSOIRE POUR STOMIE

(30) Priority: 30.06.2011 DK 201170347
(43) Date of publication of application: 07.05.2014
(73) Proprietor: Coloplast A/S, 3050 Humlebæk (DK)
(72) Inventor: KLEIN, Charlotte, 2700 Broenshoej (DK); STROEBECH, Esben, 2970 Hoersholm (DK)
(86) International application number: PCT/DK2012/050217
(87) International publication number: WO 2013/000472

(56) References cited:
- WO-A1-2009/000273
- GB-A- 2 225 956
- US-A1- 2007 005 032
- US-B1- 6 332 879

## Description

### FIELD OF THE INVENTION

The present invention relates to an ostomy appliance comprising a collecting bag and an adhesive wafer onto which a first and a second release liner are fastened. Moreover, the present invention relates to a method for adjusting an ostomy appliance to the shape of a stoma.

### BACKGROUND OF THE INVENTION

WO2009/000273 discloses an ostomy appliance for attachment to the stoma body comprising a soft adhesive wafer having a tensile strength at 20% strain of less than 0.75N/4mm and a collecting pouch attachable to the adhesive wafer, the wafer comprises a backing layer and a skin-facing adhesive layer, wherein the adhesive skin-facing surface of the wafer is provided with at least two release liners each covering at least a part of the adhesive surface and together covering the entire adhesive surface.

When an adhesive surface of a wafer of an ostomy appliance is covered by two release liners and a cutting guide is printed onto both release liners, then the requirements to aligning the two release liners correctly relative to each other are high as incorrectly aligned cutting guides may mislead the user and make it difficult to adapt a passage in the wafer which fits the shape of the stoma of the user.

It is an object of one or more embodiments of the present invention to provide a solution to this problem.

### DESCRIPTION OF THE INVENTION

In a FIRST aspect, the present invention relates to an ostomy appliance for collecting waste material from a stoma, the ostomy appliance comprising:
an adhesive wafer defining a passage for receiving a stoma of a user, the adhesive wafer comprising a backing layer and a skin facing adhesive layer; and
a collecting bag which in use is secured to the adhesive wafer;
wherein a first part of the skin facing adhesive layer is covered by a first release liner and wherein a second part of the skin facing adhesive layer is covered by a second release liner;
wherein at least one of the first and the second release liners defines an attached part which in an initial state is attached to the skin facing adhesive layer, and a foldable part which in a folded state is folded such that it is not in direct contact with the skin facing adhesive layer;
wherein a cutting guide is printed onto the at least a part of the attached part and the foldable part.

By providing a cutting guide which covers at least a part of the attached part and a part of the foldable part it is irrelevant whether the first and the second release liners are symmetrically arranged relative to each other and relative to the passage of the adhesive wafer. The reason for this is that the user prior to adapting the shape of the passage to the ostomy may bring the foldable part into its unfolded state and only use the cutting guide of the respective release liner as the cutting guide. The skilled person will readily realize that the attached part and the foldable part will always be correctly aligned relative to each other.

Moreover, it will be appreciated that in the opposite case where one part of the cutting guide is provided on one release liner while another part of the cutting guide is provided the other another release line, then it is essential that the two cutting guides are exactly aligned in order for the user to use the two parts of the cutting guide as a guiding tool. If the two release liners are not aligned, the two halves of the cutting guide will mislead more than guide the user in the process of adapting the passage to the shape of the stoma. This problem is solved by providing a foldable release liner.

The collecting bag may be suitable for collecting waste material from the intestinal system of a human being. Accordingly, the ostomy appliance may be adapted for use in connection with colostomies, ileostomies or urostomies, both temporary and permanent types. However, the appliance may also be used for collecting fecal matter from the rectum of a person suffering from fecal incontinence without having undergone stoma surgery.

The passage defined in the adhesive wafer, may be circular, oval or polygonal in shape. It will be appreciated that in some embodiments, this passage is small enough to allow the user to customize the passage to the shape of the stoma of the user (i.e. the passage must be smaller than the stoma), while at the same time being large enough to allow a cutting means such as a scissor, to be inserted into the passage. Thus, in one embodiment, the largest dimension of the passage is below 20 mm, such as below 15 mm, such as below 10 mm, such as below 5 mm,

The skin facing adhesive layer may comprise any suitable adhesive. The adhesive may comprise absorbent particles such as hydrocolloids. Preferred adhesive are soft adhesives, such as silicone or polyurethane adhesives. In one embodiment the adhesive comprises a polyalkyleneoxide polymer and organosiloxane based cross-linked adhesive system.

In one embodiment, the backing layer comprises a polymer film, coating, laminate, textile or non-woven. The backing layer is preferably a highly flexible film, being strong enough for attachment of e.g. couplings and/or pouch and for removing the device in one piece, but soft enough to follow the movements of the body.

A preferred backing layer is a polyurethane film.

Preferably, the backing layer has thermoplastic elements that enable welding of e.g. a pouch or coupling ring to the adhesive wafer. Preferred thickness of the backing layer is between 15-60 µm in order to maintain the softness of the adhesive wafer.

During use, the collecting bag is secured to the adhesive wafer. Thus in one embodiment, the ostomy appliance is a one-piece ostomy appliance in which the collecting bag is permanently secured to the adhesive wafer, by welding of by adhesive means. In the latter embodiment, the collecting bag may be integrated with the wafer. The collecting bag may be secured to the adhesive wafer by means of an adhesive provided between the collecting bag and the adhesive wafer. The latter adhesive may be provided on the opposite side of the adhesive wafer than the skin facing adhesive layer. Alternatively, or as a supplement, the collecting bag may be coupled to the adhesive wafer by means of a mechanical coupling means.

In another embodiment, the collecting bag and the adhesive form a two-piece ostomy appliance which is delivered to the user in two pieces that must be connected to each other by the user. In the latter embodiment, the adhesive may be provided on mating surfaces of the collecting bag and the wafer. In one embodiment, the collecting bag is detachably attached to the adhesive wafer, i.e. such that the collecting bag may be detached from the adhesive wafer.

In one embodiment, the collecting bag is reattachably attached to the adhesive wafer. By reattachably attached shall be understood that the collecting bag is attached or attachable to the adhesive wafer such that it may subsequently be detached and reattached to the adhesive wafer.

In one embodiment, the shape of the adhesive wafer is round such as circular or oval. The skin facing adhesive layer is covered by a first and a second release liner. Such that a first part of the skin facing adhesive layer is covered by the first part and the second part of the skin facing adhesive layer is covered by the second part.

At least one of the two release liners defines an attached part and a non-attached part. The attached part may in an initial state be attached to the skin facing adhesive layer such that this part of the skin facing adhesive layer is prevented from being contaminated. At the same time, the non-attached part may not be attached to the skin facing adhesive layer. The non-attached part may be foldable relative to the attached part such that the two parts are separated by a folding line. When the non-attached part is folded, it may be designated the foldable part. The foldable part may be folded onto the attached part when the release liner is in its folded state. By "folded onto the attached part" shall be understood that the two parts are folded into contact with each other without being attached to each other (except from the interconnecting folding line). Accordingly, the folded part is free to be moved relative to the attached part. In the initial state, the foldable part may not be in direct contact with the skin facing adhesive layer i.e. the surfaces of the foldable part do not contact the adhesive layer and is only connected to this layer via the attached part.

In one embodiment, at least a part of the folded part is attached to the attached part. As an example, a zone of the foldable part may be temporarily secured to the attached part, such that the user easily may detach the foldable part from the attached part and unfold it into its unfolded state.

It will be appreciated that when the release liner is in its initial state, it may also be in its folded state. When the release liner is in its folded state, the foldable part may cover at least a part of the attached part such as 10 percent of the attached part, such as 20 percent of the attached part, such as 30 percent of the attached part, such as 40 percent of the attached part, such as 50 percent of the attached part, such as 60 percent of the attached part, such as 70 percent of the attached part, such as 90 percent of the attached part, such as 90 percent of the attached part or such as 100 percent of the attached part. When the foldable part covers 100 percent of the attached part, the two parts may be said to coincide, e.g. such that the outer rim of the attached part coincides with the outer rim of the foldable part.

Due to the provision of the adhesive on the skin facing adhesive layer, the attached part may simply be secured to the skin facing adhesive layer by being brought into contact with the skin facing adhesive layer during manufacture such that the tackiness of the adhesive of the skin facing adhesive layer adheres to the release liner.

In one embodiment, the release liner is siliconised or otherwise provided with a non-stick surface on the side which faces the skin facing adhesive layer.

In one embodiment, the skin facing adhesive layer is covered by a plurality of release liners such as two, three, four, five or six. One or more - such as each - of these release liners may define an attached part and a foldable part. In one embodiment, one or more of the plurality of release liners only defines an attached part.

In one embodiment, the foldable part defines an unfolded state in which the foldable part and the attached part are provided in substantially the same plane. In one embodiment, the foldable part and the attached parts are defined in two parallel planes when the foldable part is in its unfolded state. In one embodiment, the two parallel planes are spaces apart with a distance which is less than five times the thickness of the release liner, such as less than three times the thickness of the release liner, such as less than twice the thickness of the release liner.

A cutting guide is printed onto the at least a part of the attached part and the foldable part. In one embodiment, the cutting guide is printed onto one side of the release liner. Alternatively, a cutting guide is printed onto both sides of the release liner. Alternatively, a cutting guide may be integrated into the release liner. In one embodiment, the cutting guide is visible from one side of the release liner. In another embodiment, a cutting guide is visible from both sides of the release liner. One advantage of the latter is that the cutting guide may be used independent of whether the foldable part is in its folded or unfolded state.

In one embodiment, the first and the second release liners are secured to a first and a second part of the skin facing adhesive layer.

The cutting guide may be round or quadrangular. As an example, the rounded shape may be circular or elliptical or oval. In one embodiment, the predetermined shapes may be concentric with respect to the passage. As an example, the passage may be circular and a plurality of concentric circles may form the cutting guide.

In one embodiment, the cutting guide comprises an indication of the distance from the centre and/or the rim of the passage to the one or more - such as each - of the predetermined shapes.

In one embodiment, the cutting guide in the unfolded state encirculates the passage. As an example the cutting guide may form one or more concentric circles which encirculate the passage. One advantage of an encirculating cutting guide is that a cutting guide need only be provided on one of the two release liners, as this release liner may be brought into its unfolded state in which the user may use the encirculating cutting guide to adapt the passage to the shape of the ostomy.

In one embodiment, the cutting guide in the unfolded state is arranged substantially symmetrically around the passage in the adhesive wafer. E.g. by being provided as concentric circles or oval shapes. Alternatively, the cutting guide may only show a part of a predetermined geometry, e.g. the edges of a quadrangle. These parts may be provided substantially symmetrically relative to the passage.

In one embodiment, the first release liner comprises an attached part and no foldable part. In this embodiment, only the second release liner forms an attached part and a foldable part. In this embodiment, the cutting guide may solely be provided on one or both of the surfaces of the second release liner.

Alternatively, each of the first and the second release liners defines an attached part and a foldable part. By providing two foldable parts, users with poor dexterity may easily remove the two release liners as the two release liners may be removed by pulling the foldable parts in opposite directions. In one embodiment, a cutting guide is provided on only one of the two foldable release liners. In another embodiment, a cutting guide is provided on both of the two foldable release liners. By providing a cutting guide on both release liners, a user may choose between the two cutting guides in accordance with the users preference e.g. due to being right or left handed.

In one embodiment, the attached parts of the first and the second release liners are arranged symmetrically on the wafer. This symmetrical arrangement may be relative to the outer rim of the wafer. Alternatively, the symmetrical arrangement may be relative to the inner rim of the wafer i.e. the inner rim of the passage.

In one embodiment, the attached parts of the first and the second release liners cover substantially an entire surface of the skin facing adhesive layer. In alternative embodiment, the first and the second release liners only covers a part of the skin facing adhesive layer, while other release liners covers the remaining parts of the skin facing adhesive layer.

In a SECOND aspect the present invention relates to a method of adjusting an ostomy appliance according to the first aspect of the invention, comprising the steps of:
- folding the foldable part of one of the first and the second release liners into the unfolded state;
- using the cutting guide to cut a hole in the adhesive wafer so as to customize the hole to the shape of a stoma of the user.

As the method may be used on an ostomy appliance according to the first aspect, it may be used on an ostomy appliance for attachment to a stoma, the ostomy appliance comprising:
an adhesive wafer defining a passage for receiving a stoma of a user, the adhesive wafer comprising a backing layer and a skin facing adhesive layer; and
a collecting bag which in use is secured to the adhesive wafer;
wherein a first part of the skin facing adhesive layer is covered by a first release liner and wherein a second part of the skin facing adhesive layer is covered by a second release liner;
wherein at least one of the first and the second release liners defines an attached part which in an initial state is attached to the skin facing adhesive layer, and a foldable part which in a folded state is folded such that it covers at least a part of the attached part and such that it is not in direct contact with the skin facing adhesive layer;
wherein a cutting guide is printed onto the at least a part of the attached part and the foldable part.

Moreover, the method may comprise the step of:
removing one of the first and the second release liners and folding the foldable part of the other the first and the second release liners onto that part of the skin facing adhesive layer from which the first or second release liner was removed.

By removing the one of the two release liners the foldable part of the other may be temporarily fastened to the skin facing adhesive layer, whereby it is secured to the adhesive layer during customization of the aperture to the shape of the stoma of the user.

In yet another embodiment, the method comprises the step of:
- removing the first and/or the second release liner so as to expose the skin facing adhesive layer.

This provides for bringing the adhesive into contact with the skin of the user, and thus the method may comprise the step of:
- bringing the skin facing adhesive layer into contact with the skin of the user.

### BRIEF DESCRIPTION OF THE FIGURES

Fig. 1 discloses a first embodiment of the adhesive wafer and the release liner,
Fig. 2 discloses a second embodiment of the adhesive wafer and the release liner,
Fig. 3 discloses a cross-sectional view of the adhesive wafer and the release liner, and
Fig. 4 discloses a cross-sectional view of the adhesive wafer according to the present invention.

### DETAILED DESCRIPTION OF THE FIGURES

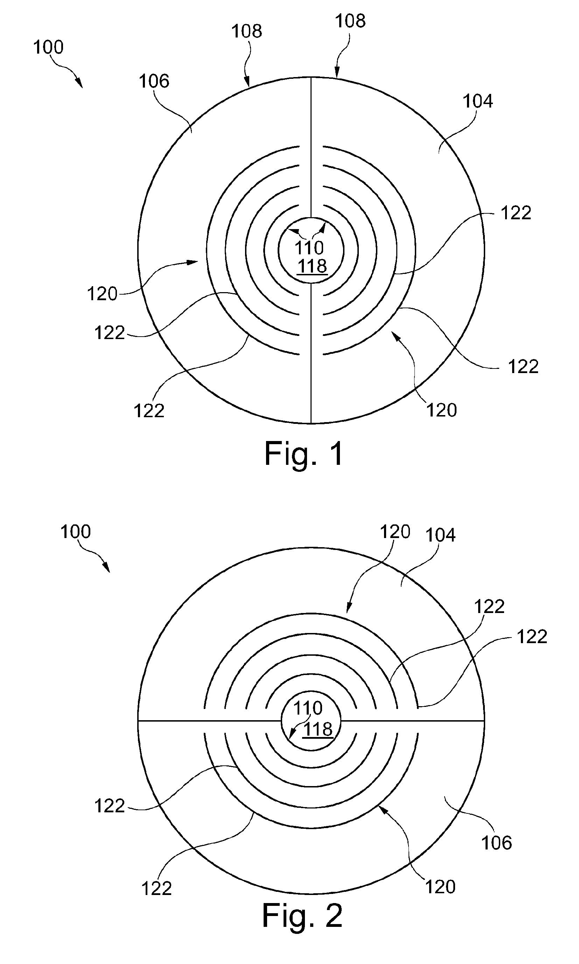

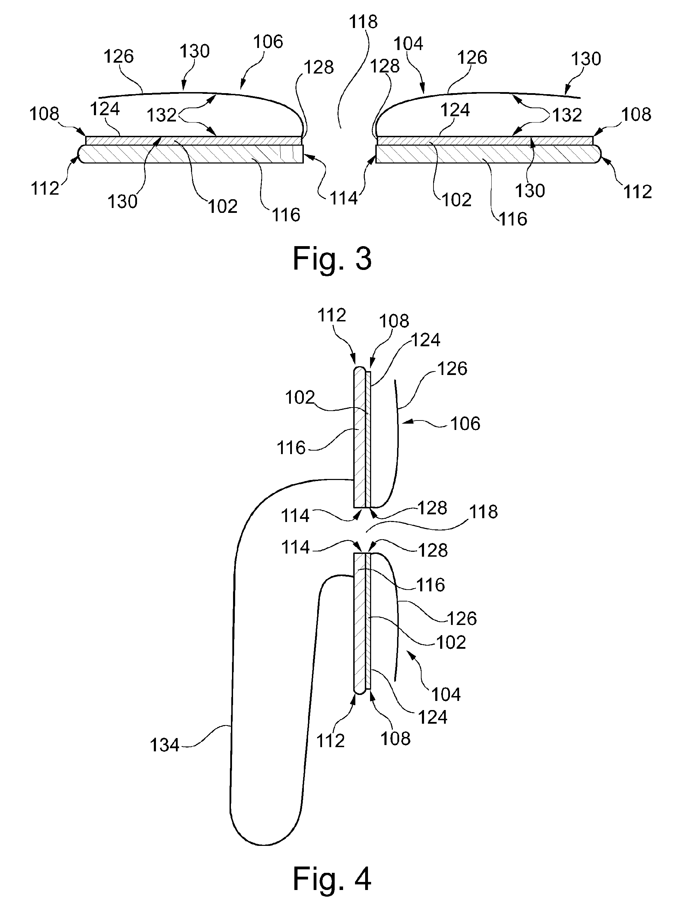

Figs. 1 and 2 disclose a rear elevational view of an adhesive wafer 100 according to the present invention. The adhesive wafer 100 comprises a skin facing adhesive layer 102 (cf. Figs. 3 and 4) onto which a first release liner 104 and a second release liner 106 are provided. The two release liners are provided such that they coincide with the surface of the skin facing adhesive layer 102. Accordingly, an outer rim 108 of each of the first and the second release liners 104,106 coincide with a corresponding outer rim 112 (see Figs. 3 and 4) of the adhesive wafer 100. Similarly, an inner rim 110 of the first release liner 104 and the second release liner 106 coincide with an inner rim 114 of the adhesive wafer 100 (see Figs. 3 and 4). The skin facing adhesive layer 102 is provided on a backing layer 116 of the adhesive wafer 100. The skin facing adhesive layer 102 is revealed when the release liners 104,106 are removed.

The adhesive wafer 100 defines a passage 118 which is encircled by the inner rim 114. Normally this passage 118 does not fit the outer geometry of the stoma of the user. Thus, the user must adjust the shape of the passage 118 to the shape of the stoma. In order to do this, at least one of the first and the second release liners 104,106 comprises a cutting guide 120. The cutting guide 120 may comprise one or more concentric lines 122 each of which is concentric with respect to the passage 118. In the embodiment of Figs. 1 and 2 the four concentric lines 122 are provided.

In the embodiments of the figures, each of the first release liner 104 and the second release liner 106 defines an attached part 124 and a foldable part 126. The attached part 124 is secured to the skin facing adhesive layer 102 due to the tackiness of the skin facing adhesive layer 102. The foldable part 126 is separated from the attached part 124 by the folding line 128. The foldable part 126 is foldable between a folded state and an unfolded state. In the folded state, the foldable part 126 is folded into contact with the attached part 124. In the unfolded state the foldable part 126 is folded away from the attached part 124. When the foldable part 126 is folded away from its own attached part 124, the foldable part may be folded into contact with another release liner. As an example, the foldable part 126 of the first release liner 104 may be folded into contact with the second release liner 106.

The cutting guide 120 may be printed onto the first side 130 or the second side 132 of the release liner. In the figures, the first side 130 is that side of the release liner 104,106 which is in direct contact with the skin facing adhesive layer 102. It will be appreciated that only the first side of the attached part 124 is in direct contact with the skin facing adhesive layer 102 while the first side of the foldable part 126 faces away from the adhesive wafer 100 in the embodiments of the figure.

It will be appreciated that when the cutting guide 120 is provided on the second side 132, the entire cutting guide 120 is visible when the foldable part 126 is folded into its unfolded position. In this position the cutting guide 120 (a part of which is provided on the attached part 124 and a part of which is provided on the foldable part 126) may be used to adjust the shape of the passage 118 such that it fits the geometry of the stoma of the user.

In the embodiment of Fig. 4, the collecting bag 134 is provided in a form where it is permanently secured to the adhesive wafer 100, whereby the ostomy appliance of Fig. 4 is a so-called one piece ostomy appliance. In other embodiments, the collecting bay is adapted to be secured to the adhesive wafer 100 by the user, e.g. by means of an adhesive. In the latter case, the ostomy appliance is a so-called two-piece appliance.

## Claims

1. An ostomy appliance for attachment to a stoma, the ostomy appliance comprising:
an adhesive wafer (100) defining a passage (118) for receiving a stoma of a user, the adhesive wafer (100) comprising a backing layer (116) and a skin facing adhesive layer (102); and
a collecting bag (134) which in use is secured to the adhesive wafer (100);
wherein a first part of the skin facing adhesive layer (102) is covered by a first release liner (104) and wherein a second part of the skin facing adhesive layer (102) is covered by a second release liner (106);
wherein at least one of the first and the second release liners (104,106) defines an attached part (124) which in an initial state is attached to the skin facing adhesive layer (102), and a foldable part (126) which in a folded state is folded such that it is not in direct contact with the skin facing adhesive layer (102);
**characterized in that** a cutting guide (120) is printed onto the at least a part of the attached part (124) and the foldable part (126).

2. An ostomy appliance according to claim 1, wherein the foldable part (126) defines an unfolded state in which the foldable part (126) and the attached part (124) are provided in substantially the same plane.

3. An ostomy appliance according to claim 1 or 2, wherein the cutting guide (120) in the unfolded state encirculates the passage (118).

4. An ostomy appliance according to any of the preceding claims, wherein the cutting guide (120) in the unfolded state is arranged substantially symmetrically around the passage (118) in the adhesive wafer (100).

5. An ostomy appliance according to any of the preceding claims, wherein the first release liner (104) comprises an attached part (124) and no foldable part.

6. An ostomy appliance according to any of claims 1-4, wherein each of the first and the second release liners (104, 106) defines an attached part (124) and a foldable part (126).

7. An ostomy appliance according to any of the preceding claims, wherein the attached parts (124) of the first and the second release liners (104, 106) are arranged symmetrically on the wafer (100).

8. An ostomy appliance according to any of the preceding claims, wherein the attached parts (124) of the first and the second release liners (104, 106) cover substantially an entire surface of the skin facing adhesive layer (102).

9. An ostomy appliance according to any of the preceding claims, wherein the collecting bag (134) is reattachably attached to the adhesive wafer (100).

10. An ostomy appliance according to any of claims 1-9, wherein the collecting bag (134) is integrated with the wafer (100).

11. A method of adjusting an ostomy appliance according to any of the preceding claims, comprising the steps of:
- folding the foldable part (126) of one of the first and the second release liners (104, 106) into the unfolded state;
- using the cutting guide (120) to cut a hole in the adhesive wafer (100) so as to customize the hole to the shape of a stoma of the user.

## Patentansprüche

1. Stomavorrichtung zum Befestigen an einem Stoma, wobei die Stomavorrichtung umfasst:
eine Haftscheibe (100), die einen Durchgang (118) zum Aufnehmen eines Stomas eines Benutzers definiert, wobei die Haftscheibe (100) eine Trägerschicht (116) und eine zu der Haut zeigende Haftschicht (102) umfasst; und
einen Sammelbeutel (134), der in Verwendung an der Haftscheibe (100) befestigt ist;
wobei ein erster Teil der zu der Haut zeigenden Haftschicht (102) von einer ersten Abziehfolie (104) bedeckt ist und wobei ein zweiter Teil der zu der Haut zeigenden Haftschicht (102) von einer zweiten Abziehfolie (106) bedeckt ist;
wobei wenigstens eine von der ersten und der zweiten Abziehfolie (104, 106) einen befestigten Teil (124) definiert, der in einem Anfangsstadium an der zu der Haut zeigenden Haftschicht (102) befestigt ist, und einen faltbaren Teil (126), der sich in einem gefalteten Zustand befindet, so dass er nicht in direktem Kontakt mit der zu der Haut zeigenden Haftschicht (102) steht;
**dadurch gekennzeichnet, dass** eine Schneideführung (120) auf wenigstens einen Teil des befestigten Teils (124) und des faltbaren Teils (126) gedruckt ist.

2. Stomavorrichtung gemäß Anspruch 1, wobei der faltbare Teil (126) einen entfalteten Zustand definiert, bei dem der faltbare Teil (126) und der befestigte Teil (124) im Wesentlichen in der gleichen Ebene angeordnet sind.

3. Stomavorrichtung gemäß Anspruch 1 oder 2, wobei die Schneideführung (120) in dem entfalteten Zustand den Durchgang (118) umgibt.

4. Stomavorrichtung gemäß einem der vorstehenden Ansprüche, wobei die Schneideführung (120) in dem entfalteten Zustand im Wesentlichen symmetrisch um den Durchgang (118) in der Haftscheibe (100) angeordnet ist.

5. Stomavorrichtung gemäß einem der vorstehenden Ansprüche, wobei die erste Abziehfolie (104) einen befestigten Teil (124) und keinen faltbaren Teil umfasst.

6. Stomavorrichtung gemäß einem der Ansprüche 1-4, wobei jede von der ersten und der zweiten Abziehfolie (104, 106) einen befestigten Teil (124) und einen faltbaren Teil (126) definiert.

7. Stomavorrichtung gemäß einem der vorstehenden Ansprüche, wobei die befestigten Teile (124) der ersten und der zweiten Abziehfolie (104, 106) symmetrisch auf der Scheibe (100) angeordnet sind.

8. Stomavorrichtung gemäß einem der vorstehenden Ansprüche, wobei die befestigten Teile (124) der ersten und der zweiten Abziehfolie (104, 106) im Wesentlichen die gesamte Oberfläche der zu der Haut zeigenden Haftschicht (102) bedecken.

9. Stomavorrichtung gemäß einem der vorstehenden Ansprüche, wobei der Sammelbeutel (134) wiederbefestigbar an der Haftscheibe (100) befestigt ist.

10. Stomavorrichtung gemäß einem der Ansprüche 1-9, wobei der Sammelbeutel (134) mit der Scheibe (100) integriert ist.

11. Verfahren zum Anpassen einer Stomavorrichtung gemäß einem der vorstehenden Ansprüche, umfassend die Schritte:
- Falten des faltbaren Teils (126) von einer von der ersten und der zweiten Abziehfolie (104, 106) in den entfalteten Zustand;
- Verwenden der Schneideführung (120) zum Schneiden eines Lochs in die Haftscheibe (100), um das Loch an die Form eines Stomas des Benutzers anzupassen.

## Revendications

1. Accessoire pour stomie à fixer à une bouche, l'accessoire pour stomie comprenant:
une galette adhésive (100) qui définit un passage (118) pour recevoir une bouche d'un utilisateur, la galette adhésive (100) comprenant une couche de support (116) et une couche adhésive opposée à la peau (102); et
un sac de collecte (134) qui, lors de l'utilisation, est fixé à la galette adhésive (100);
dans lequel une première partie de la couche adhésive opposée à la peau (102) est recouverte par une première garniture amovible (104) et dans lequel une deuxième partie de la couche adhésive opposée à la peau (102) est recouverte par une deuxième garniture amovible (106);
dans lequel au moins une des première et deuxième garnitures amovibles (104, 106) définit une partie attachée (124) qui dans un état initial est attachée à la couche adhésive opposée à la peau (102), et une partie pliable (126) qui dans un état plié est pliée de telle sorte qu'elle ne soit pas en contact direct avec la couche adhésive opposée à la peau (102);
**caractérisé en ce qu'**un guide de coupe (120) est imprimé sur ladite au moins une partie parmi la partie attachée (124) et la partie pliable (126).

2. Accessoire pour stomie selon la revendication 1, dans lequel la partie pliable (126) définit un état déplié dans lequel la partie pliable (126) et la partie attachée (124) se trouvent sensiblement dans le même plan.

3. Accessoire pour stomie selon la revendication 1 ou 2, dans lequel le guide de coupe (120) dans l'état déplié encercle le passage (118).

4. Accessoire pour stomie selon l'une quelconque des revendications précédentes, dans lequel le guide de coupe (120) dans l'état déplié est agencé de façon sensiblement symétrique autour du passage (118) dans la galette adhésive (100).

5. Accessoire pour stomie selon l'une quelconque des revendications précédentes, dans lequel la première garniture amovible (104) comprend une partie attachée (124) et une partie non pliable.

6. Accessoire pour stomie selon l'une quelconque des revendications 1 à 4, dans lequel chacune des première et deuxième garnitures amovibles (104, 106) définit une partie attachée (124) et une partie pliable (126).

7. Accessoire pour stomie selon l'une quelconque des revendications précédentes, dans lequel les parties attachées (124) des première et deuxième garnitures amovibles (104, 106) sont agencées de façon symétrique sur la galette (100).

8. Accessoire pour stomie selon l'une quelconque des revendications précédentes, dans lequel les parties attachées (124) des première et deuxième garnitures amovibles (104, 106) couvrent sensiblement la totalité d'une surface de la couche adhésive opposée à la peau (102).

9. Accessoire pour stomie selon l'une quelconque des revendications précédentes, dans lequel le sac de collecte (134) est attaché de façon rattachable à la galette adhésive (100).

10. Accessoire pour stomie selon l'une quelconque des revendications 1 à 9, dans lequel le sac de collecte (134) est intégré à la galette (100).

11. Procédé d'ajustement d'un accessoire pour stomie selon l'une quelconque des revendications précédentes, comprenant les étapes suivantes:
- plier la partie pliable (126) de l'une des première et deuxième garnitures amovibles (104, 106) dans l'état déplié; et
- utiliser le guide de coupe (120) pour découper un trou dans la galette adhésive (100) de manière à adapter le trou à la forme d'une bouche de l'utilisateur.
